(19) **Europäisches Patentamt European Patent Office Office européen des brevets**

(11) **EP 4 661 019 A1**

(12) **EUROPEAN PATENT APPLICATION**
published in accordance with Art. 153(4) EPC

(43) Date of publication:
**10.12.2025 Bulletin 2025/50**

(51) International Patent Classification (IPC):
**G16H 20/40** (2018.01)

(21) Application number: **24773754.7**

(22) Date of filing: **05.01.2024**

(52) Cooperative Patent Classification (CPC):
**G06N 3/04; G06N 3/08; G06T 17/00; G06V 10/44; G06V 10/82; G16H 20/40**

(86) International application number:
**PCT/CN2024/070704**

(87) International publication number:
**WO 2024/193184 (26.09.2024 Gazette 2024/39)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **17.03.2023 CN 202310277453**

(71) Applicant: **Hangzhou Chohotech Co., Ltd.**
**Hangzhou, Zhejiang 310023 (CN)**

(72) Inventors:
• **SONG, Yiqun**
  **Hangzhou, Zhejiang 310023 (CN)**
• **ZHAO, Zeyu**
  **Hangzhou, Zhejiang 310023 (CN)**

(74) Representative: **Gille Hrabal**
**Partnerschaftsgesellschaft mbB**
**Patentanwälte**
**Brucknerstraße 20**
**40593 Düsseldorf (DE)**

(54) **COMPUTER-IMPLEMENTED METHOD FOR GENERATING ORTHODONTIC TOOTH TREATMENT SCHEME**

(57) The present application provides a computer-implemented method for generating an orthodontic treatment plan, the method comprises: obtaining a first and a second 3D digital models, where the first 3D digital model represents an initial tooth arrangement of a jaw/jaws, and the second 3D digital model represents a target tooth arrangement of the jaw/jaws; extracting features from the first 3D digital model using a trained feature extraction deep neural network; and generating an orthodontic treatment plan of the jaw/jaws using a trained multi-agent reinforcement learning based deep neural network based on the extracted features and the second 3D digital model, where in the multi-agent reinforcement learning based deep neural network, each tooth is taken as an agent, where the orthodontic treatment plan utilizes shell-shaped tooth repositioners and comprises a series of successive treatment steps to incrementally reposition the jaw/jaws from the initial tooth arrangement to the target tooth arrangement.

100

a first and a second 3D digital models are obtained — 101

features are extracted from the first 3D digital model — 103

an orthodontic treatment plan is generated by a multi-agent reinforcement learning based deep neural network based on the extracted features — 105

**FIG. 1**

**Description**

**FIELD OF THE APPLICATION**

The present application generally relates to a computer-implemented method for generating an orthodontic treatment plan.

**BACKGROUND**

Nowadays, shell-shaped tooth repositioners made of polymer materials become more and more popular due to their advantages on aesthetic appearance, convenience and hygiene. As for orthodontic treatment of teeth of a single jaw (upper jaw or lower jaw), a set of shell-shaped tooth repositioners usually comprises a dozen of, even tens of successive shell-shaped tooth repositioners for incrementally repositioning the teeth from an initial tooth arrangement to a target tooth arrangement, where between the initial tooth arrangement and the target tooth arrangement, there are N successive intermediate tooth arrangements from a first intermediate tooth arrangement to a last intermediate tooth arrangement.

The series of successive shell-shaped tooth repositioners are made based on 3D digital models representing the series of successive tooth arrangements, respectively. The series of successive tooth arrangements are usually referred to as an orthodontic treatment plan of the jaw, which specifies an order of movement for the teeth, including an order of different movements of each tooth.

Usually, the orthodontic treatment plan for the teeth of a single jaw is generated based on the initial tooth arrangement and the target tooth arrangement of the teeth. At present, this process requires manual intervention, and professionals that design orthodontic treatment plans are required to undergo extensive relevant trainings. Since manual intervention is required, the efficiency of the process is low, and the cost of the process is high.

In view of the above, it is necessary to provide a new method for generating an orthodontic treatment plan.

**SUMMARY**

In one aspect, the present application provides a computer-implemented method for generating an orthodontic treatment plan, the method comprises: obtaining a first 3D digital model and a second 3D digital model, where the first 3D digital model represents an initial tooth arrangement of a jaw/jaws, and the second 3D digital model represents a target tooth arrangement of the jaw/jaws; extracting features from the first 3D digital model using a trained feature extraction deep neural network; and generating an orthodontic treatment plan of the jaw/jaws using a trained multi-agent reinforcement learning based deep neural network based on the extracted features and the second 3D digital model, where in the multi-agent reinforcement learning based deep neural network, each tooth of the jaw/jaws is taken as an agent, where the orthodontic treatment plan utilizes shell-shaped tooth repositioners and comprises a series of successive treatment steps to incrementally reposition the jaw/jaws from the initial tooth arrangement to the target tooth arrangement.

In some embodiments, the computer-implemented method for generating an orthodontic treatment plan may further comprise: calculating displacements of single movements of the teeth based on the first 3D digital model and the second 3D digital model, the multi-agent reinforcement learning based deep neural network generating the orthodontic treatment plan of the jaw/jaws based on the extracted features, the second 3D digital model and the calculated displacements of single movements of the teeth.

In some embodiments, the jaws may comprise teeth of an upper jaw and a lower jaw.

In some embodiments, the computer-implemented method for generating an orthodontic treatment plan may further comprise: uniformly sampling M points from each tooth of the first 3D digital model to obtain a point cloud X of the jaw/jaws, the feature extraction deep neural network extracting features from the point cloud X.

In some embodiments, the feature extraction deep neural network may comprise a first MLP module and a second MLP module, where the first MLP module is configured to extract the features, and the second MLP module is configured to reconstruct the point cloud of the jaw/jaws based on the features extracted by the first MLP module in the training of the feature extraction deep neural network.

In some embodiments, the multi-agent reinforcement learning based deep neural network may be one of the following networks: a recurrent network, a CommNet and a G2ANet.

In some embodiments, the multi-agent reinforcement learning based deep neural network may be modeled using one of the following algorithms: Qmix, VDN, COMA and QTRAIN.

In some embodiments, in each frame, actions selectable by each agent may comprise: maintaining still, translating once only, rotating once only, and translating once and rotating once.

In some embodiments, constraints imposed by the multi-agent reinforcement learning based deep neural network on the agents may comprise: a constraint on displacements single movements, a constraint on collision, a constraint on movement separation and a constraint on anchorage.

In some embodiments, the multi-agent reinforcement learning based deep neural network may assign a reward to each frame according to the following: whether an agent violates any constraint, whether an agent selects translation or rotation, whether an agent reaches its target pose, and whether all agents reach their target poses.

In some embodiments, the orthodontic treatment plan may be a tooth action chart comprising an action taken by each tooth in each treatment step in the process of repositioning the jaw/jaws from the initial tooth arrangement to the target tooth arrangement.

In another aspect, the present application provides a computer system for generating an orthodontic treatment plan, it comprises a processor and a storage device, where the storage device stores therein a computer program for generating the orthodontic treatment plan, which computer program, when executed, will cause the processor to implement the method for generating an orthodontic treatment plan.

## BRIEF DESCRIPTION OF THE DRAWINGS

The above and other features of the present application will be further illustrated below with reference to figures and their detailed description. It should be appreciated that these figures only show several exemplary embodiments according to the present application, so they should not be construed as limiting the scope of the present application. Unless otherwise specified, the figures are not necessarily drawn to scale, and similar reference numbers therein denote similar components.

FIG. 1 schematically illustrates a flow chart of a computer-implemented method for generating an orthodontic treatment plan according to one embodiment of the present application;

FIG. 2 schematically illustrates a basic structure of a feature extraction deep neural network according to one embodiment of the present application;

FIG. 3 schematically illustrates a basic structure of a multi-agent reinforcement learning based deep neural network according to one embodiment of the present application.

## DETAILED DESCRIPTION OF ILLUSTRATED EMBODIMENTS

In the following detailed description, reference is made to the accompanying drawings, which form a part thereof. Exemplary embodiments in the detailed description and figures are only intended for illustration purpose and not meant to be limiting. Inspired by the present application, those skilled in the art can understand that other embodiments may be utilized and other changes may be made, without departing from the spirit or scope of the present application. It will be readily understood that aspects of the present application described and illustrated herein can be arranged, replaced, combined, separated and designed in a wide variety of different configurations, all of which are explicitly contemplated and make part of the present application.

One aspect of the present application provides a computer-implemented method for generating an orthodontic treatment plan, which method automatically generates an orthodontic treatment plan using a multi-agent reinforcement learning based deep neural network based on a 3D digital model representing an initial tooth arrangement of a jaw/jaws and a 3D digital model representing a target tooth arrangement of the jaw/jaws. In one embodiment, the orthodontic treatment plan may be a tooth action chart comprising an action taken by each tooth in each treatment step in the process of repositioning the jaw/jaws from the initial tooth arrangement to the target tooth arrangement. It is understood that a series of successive intermediate tooth arrangements between the initial tooth arrangement and the target tooth arrangement can be generated based on the tooth action chart and the initial tooth arrangement or the target tooth arrangement, and the orthodontic treatment plan may also be the series of successive intermediate tooth arrangements. It is understood that 3D digital models respectively representing the series of successive intermediate tooth arrangements between the initial tooth arrangement and the target tooth arrangement can be generated based on the 3D digital model representing the initial tooth arrangement or the target tooth arrangement and the series of successive intermediate tooth arrangements, and the orthodontic treatment plan may also be the 3D digital models representing the series of successive intermediate tooth arrangements, respectively.

Another aspect of the present application provides a computer system for generating an orthodontic treatment plan, which computer system comprises a processor and a storage device, where the storage device stores therein a computer program for generating an orthodontic treatment plan, the computer program, when executed, will cause the processor to implement the method for generating an orthodontic treatment plan.

An orthodontic treatment is a process of repositioning teeth from an initial tooth arrangement to a target tooth arrangement. An orthodontic treatment plan comprises an order of movement of the teeth in the process of repositioning the teeth from the initial tooth arrangement to the target tooth arrangement. It is understood that the target tooth arrangement is a tooth arrangement to be achieved by the orthodontic treatment, the initial tooth arrangement may be the patient's tooth arrangement before the orthodontic treatment or the patient's current tooth arrangement based on which the orthodontic treatment plan is generated by the method of the present application.

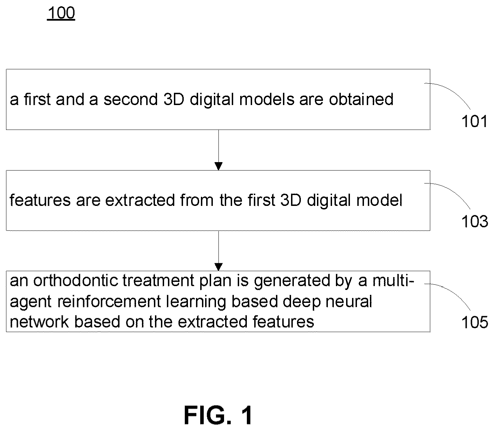

Please refer to FIG. 1, it illustrates a schematic flow chart of a computer-implemented method 100 for generating an orthodontic treatment plan according to one embodiment of the present application.

In one embodiment, the method of the present application may be used to generate an orthodontic treatment plan for teeth of an upper jaw and a lower jaw. In another embodiment, the method of the present application may be used to generate an orthodontic treatment plan for teeth of a single jaw. The method of the present application will be described in detail by an example in which an orthodontic treatment plan is generated for teeth of an upper jaw and a lower jaw.

In 101, a first 3D digital model and a second 3D digital model are obtained.

The first 3D digital model represents an initial tooth arrangement of teeth of an upper jaw and a lower jaw, and the second 3D digital model represents a target tooth arrangement of the teeth.

In one embodiment, the 3D digital model representing the initial tooth arrangement of the teeth may be obtained by directly scanning a patient's jaws. In another embodiment, the 3D digital model representing the initial tooth arrangement of the teeth may be obtained by scanning a physical model such as a plaster model of the patient's jaws. In a further embodiment, the 3D digital model representing the initial tooth arrangement of the teeth may be obtained by scanning an impression of the patient's jaws.

In one embodiment, after the 3D digital model representing the initial tooth arrangement of the teeth is obtained, the 3D digital model may be segmented to separate the teeth in the 3D digital model from one another so that each tooth in the 3D digital model may be repositioned individually.

In one embodiment, the 3D digital model representing the target tooth arrangement may be obtained based on the segmented 3D digital model representing the initial tooth arrangement of the teeth. In one embodiment, the 3D digital model representing the target tooth arrangement may be obtained by manually manipulating the segmented 3D digital model representing the initial tooth arrangement to reposition the teeth to their target poses. A pose of a tooth includes position and orientation of the tooth. In a further embodiment, the 3D digital model representing the target tooth arrangement may be obtained by automatically repositioning the teeth of the segmented 3D digital model representing the initial tooth arrangement to their target poses using a computer.

In 103, feature extraction is performed on the first 3D digital model.

Since the 3D digital model of each tooth has a large number of facets which might reach tens of thousands or even hundreds of thousands, the data volume is too big if these facets are directly used as the features of the tooth. Therefore, features of the 3D digital model in a high-dimensional space may be extracted to represent geometry information of the tooth.

In one embodiment, for each tooth of the first 3D digital model, M points are uniformly sampled from a mesh of the tooth to obtain a point cloud X of the 3D digital model. Then, N*d dimensional global features may be extracted using a trained feature extraction deep neural network based on the point cloud X. M is a predetermined number. In one embodiment, M may be determined depending on specific situations and demands, for example, M may be 4000. N is the number of teeth in the first 3D digital model.

In one embodiment, the feature extraction deep neural network may be a network based on Multilayer Perceptron (abbreviated as MLP).

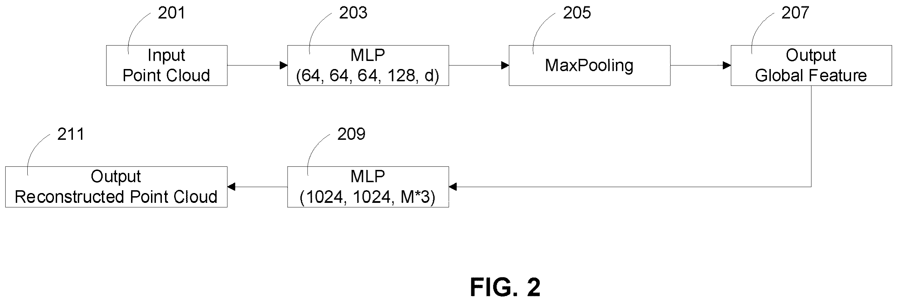

Please referring to FIG. 2, it schematically illustrates a basic structure of a feature extraction deep neural network 200 according to one embodiment of the present application.

Feature extraction deep neural network 200 comprises an input module 201, a first MLP module 203, a max pooling layer 205, a first output module 207, a second MLP module 209 and a second output module 211.

Input module 201 inputs the point cloud X to first MLP module 203. For each tooth, first MLP module 203 extracts the M*d dimensional features based on the point cloud X. Then, the max pooling layer 205 performs max pooling on the M*d dimensional features of all teeth to obtain the N*d dimensional global features. The N*d dimensional global features are output by first output module 207, and are taken as input data of subsequent generation of an orthodontic treatment plan.

In the training of feature extraction deep neural network 200, a point cloud Y is reconstructed using the second MLP module 209 based on the N*d dimensional global features, and parameters of first deep neural network 200 are obtained by iterative training with a Chamfer Distance between the two point clouds X and Y as a loss function. In one embodiment, the loss function may be written as the following Equation (1):

$$Loss = d_{CD}(X, Y) = \sum_{x \in X} \min_{y \in Y} \|x - y\|_2^2 + \sum_{y \in Y} \min_{x \in X} \|x - y\|_2^2 \qquad \text{Equation (1)}$$

In 105, an orthodontic treatment plan is generated using a trained multi-agent reinforcement leaning based deep neural network based on the global features.

The orthodontic treatment plan using shell-shaped tooth repositioners comprises a series of successive tooth arrangements. Each of the tooth arrangements may also be referred to as a frame, and represents the target tooth arrangement to be achieved by a corresponding treatment step, i.e., target poses of the teeth to be achieved by the treatment step. A pose of a tooth comprises position information and orientation information of the tooth.

In the method of the present application, each tooth is taken as an agent, and the orthodontic treatment plan is generated using a multi-agent reinforcement learning method.

In one embodiment, in each frame, each agent may select one of the following actions: maintaining still, translating once only, rotating once only, and translating once and rotating once. Then, the problem to be solved by the multi-agent reinforcement learning method is how to reasonably select an action of each agent in each frame, so that all agents satisfy predetermined constraints and the total number of the frames is as small as possible. In one embodiment, in addition to the above conditions to be satisfied, if possible, an agent may move continuously to make the repositioning path as aesthetic as possible.

In one embodiment, the following constraints may be set for the repositioning of the teeth.

A constraint on displacement of a single movement: each component of six degrees of freedom (three translation degrees of freedom and three rotation degrees of freedom) of a tooth' movement and a total displacement of a tooth' movement between two frames cannot exceed respective thresholds, i.e., a translation threshold and a rotation threshold;

A constraint on collision: in each frame, a collision amount between the teeth cannot exceed a collision threshold;

A constraint on movement separation: extrusion and torsion cannot be performed simultaneously, and retraction and intrusion cannot be performed simultaneously;

A constraint on anchorage: since for each action, there is an equal and opposite reaction, a tooth to be repositioned requires enough support to apply a repositioning force thereon.

It is understood setting of constraints is not limited to the above example, and constraints may be set according to specific demands and situations.

A core idea of reinforcement leaning is "trial-and-error": it is optimized based on feedback information obtained from iterative interactions between an agent and the environment. In the field of multi-agent reinforcement learning, the problem to be solved is typically described as a Markov decision process.

In one embodiment, this problem may be described using a partially observable Markov decision process ($S, N, O, U, T, P, R, \Upsilon$).

$N$ stands for the number of the agents.

$S$ stands for a finite set of state, and a state s of the current frame, $s = [f_0, f_1, \cdots, f_{N-1}] \in S$, is a vector, where $f_i$ stands for features of the $i^{th}$ agent, comprising information such as the d dimensional vector representing the geometry information of the agent extracted in the previous step, the current pose of the agent, and displacement of single movements of the agent (i.e., step size of each translation or rotation of the agent).

In one embodiment, displacements of single movements of an agent may be calculated based on a pose difference of a corresponding tooth between the first 3D digital model and second 3D digital model, a threshold of single translation of the tooth, and a threshold of single rotation of the tooth. For example, given the threshold of single translation of a tooth is set to 0.2 mm, and if the pose difference of the tooth between the first 3D digital model and second 3D digital model is 1.1 mm, the number of steps may be minimized on the premise that the displacement of a single translation does not exceed the threshold. In this example, the displacement of a single translation of the tooth is 1.1/6=0.18 mm.

In a further embodiment, same displacements of single movements may be set for all teeth, for example, a displacement of a single translation is set to 0.2 mm. The tooth is repositioned with the given displacements of single movements. The tooth might reach a pose close to its target pose. If the difference between the two poses is smaller than a given threshold, it may be considered that the tooth has reached the target pose.

$O$ stands for a finite set of observation, and $o_i = [g_0, g_1, \cdots, g_5] \in O$ stands for the observation of the $i^{th}$ agent in the current frame, where $g_0, \cdots, g_5$ represents features of six neighbors of the agent (itself, one left neighbor, one right neighbor, and three neighbors on the opposite jaw), which features comprise information such as d dimensional vectors representing the geometry information of the agent extracted in the previous step, the current pose of the agent, and the displacements of single movements of the agent etc.

$U$ stands for a joint action set, $u_i \in U$ stands for the action (i.e., one of the above four types of actions) of the $i^{th}$ agent in the current frame, and the selecting of the action is constrained by the constraint on movement separation described earlier.

T stands for a trajectory of the current Monte Carlo sampling.

P stands for a state transition equation.

R stands for a reward function. In the current frame s, after the agents select the joint action $u$, a reward $R(s, u)$ is given.

In one embodiment, the reward may be set as follows:

1) If any agent violates any constraint, terminate the current Monte Carlo sampling and give a reward $R(s, u) = 0$;

2) If an agent performs one translation or rotation, give a reward $R(s, u) = 1$;

3) If an agent translates or rotates continuously (translation and rotation are calculated separately), give a reward $(s, u) = 0.01$;

4) If an agent reaches its target pose, give a reward $R(s, u) = 10$;

5) If all agents reach respective target poses, terminate the current Monte Carlo sampling and assign a reward $R(s, u) = 200$.

After the reward to the current frame is calculated, a normalization operation may be performed on the reward according to the following Equation (2):

$$R(s, u) = \frac{R(S, u)}{R_{max}} \times R_{scale} \qquad \text{Equation (2),}$$

where $R_{max}$ stands for a maximum possible value of the reward, and $R_{scale}$ is a normalization factor.

$\gamma$ stands for a discount factor, and $\gamma \in [0, 1]$.

After the problem is modeled, the problem may be solved using an algorithm in the field of multi-agent reinforcement learning, for example, using a QMix algorithm. It is understood that besides QMix algorithm, any other suitable algorithm such as VDN, COMA or QTRAIN may be used instead.

In one embodiment, as for the observation of the agent i, a Recurrent Neural Network may be used to estimate $Q_i(s, a)$ function of the agent i under each possible action, where a stands for an action. In one embodiment, a Gated Recurrent Unit (abbreviated as GRU, a type of recurrent neural network) may be used to estimate the $Q_i(s, a)$ function of the agent i under each possible action.

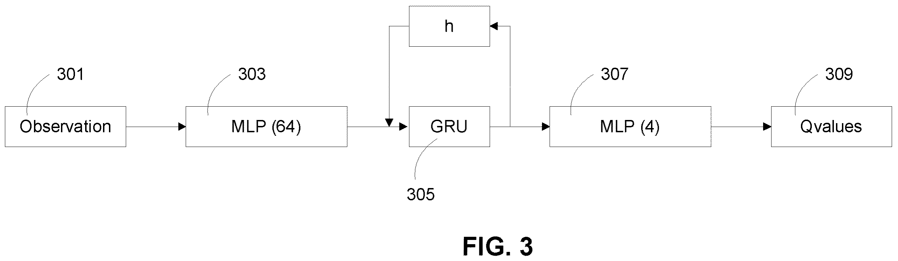

Please refer to FIG. 3, it schematically illustrates a basic structure of a multi-agent reinforcement learning based deep neural network 300 according to one embodiment of the present application.

Multi-agent reinforcement learning based deep neural network 300 comprises an observation module 301, a first MLP module 303, a GRU module 305, a second MLP module 307 and an output module 309.

Observation module 301 is the agents' observation of the environment, and is taken as input of the network to predict actions in the next step. First MLP module 303 reduces the dimensionality of input features to decrease the complexity of GRU module 305. GRU module 305 is used to estimate the $Q_i(s, a)$ function of the agent i under each possible action. The module h returns the output of the GRU module and input it to the GRU module. Second MLP module 307 is used to perform regression prediction on the output of GRU module 305 to obtain the value of each action.

After all $Q_i$ are obtained, a linear function may be used to estimate a global $Q_{tot}$ function which may be written as the following Equation (3):

$$Q_{tot}(s, a) = W[Q_0, Q_1, \cdots, Q_{N-1}]^T + b \qquad \text{Equation (3),}$$

where W and b in the Equation (3) may be predicted by first MLP module 303 and second MLP module 307, respectively, and T stands for matrix transpose.

$$TD_{target} = R(s, a) + \gamma \, Q_{tot}(s, a) \qquad \text{Equation (4)}$$

where in Equation (4), $TD_{target}$ stands for a target, and $\gamma$ stands for the discount factor.

A loss function of multi-agent reinforcement learning based deep neural network 300 may be written as the following Equation (5):

$$Loss = \sum \left( TD_{target} - Q'_{tot}(s, a) \right)^2 \qquad \text{Equation (5)}$$

In the training of multi-agent reinforcement learning based deep neural network 300, two identical networks are used, where one generates $Q_{tot}$, and the other generates $Q'_{tot}$. The difference between the two networks lies in that the parameters of the network for generating $Q_{tot}$ lag behind those of the network for generating $Q'_{tot}$ by several frames.

A series of agent actions trajectories are obtained after extensive Monte Carlo sampling, and are put in an experience pool. Some trajectories are randomly sampled from the experience pool each time to obtain the parameters of the desired model.

Lastly, value functions $Q_{tot}(s,a)$ for all possible combinations of all agents' selections of actions are derived through reasoning, and the action a corresponding to the largest value of the value function is taken as a decision value, to obtain a frame of the orthodontic treatment plan.

Inspired by the present application, it is understood that in addition to recurrent network, any other suitable neural networks such as CommNet and G2ANet may be used to generate the orthodontic treatment plan instead.

Inspired by the present application, it is understood that in addition to the above modeling method, any other suitable modeling method may be used instead, for example, other suitable reward functions may be used, or other suitable state and observation designs may be used.

The method for generating the orthodontic treatment plan according to the present application is automatically implemented by a computer, and is able to save a lot of manpower.

While various aspects and embodiments have been disclosed herein, other aspects and embodiments will be apparent to those skilled in the art, inspired by the present application. The various aspects and embodiments disclosed herein are for illustration only and are not intended to be limiting, and the scope and spirit of the present application shall be defined by the following claims.

Likewise, the various diagrams may depict exemplary architectures or other configurations of the disclosed methods and systems, which are helpful for understanding the features and functions that can be included in the disclosed methods and systems. The claimed invention is not restricted to the illustrated exemplary architectures or configurations, and desired features can be achieved using a variety of alternative architectures and configurations. Additionally, with regard to flow diagrams, functional descriptions and method claims, the order in which the blocks are presented herein shall not mandate that various embodiments of the functions shall be implemented in the same order unless otherwise the context specifies.

Unless otherwise specifically specified, terms and phrases and their variants used herein are generally intended as "open" terms instead of limiting. In some embodiments, use of phrases such as "one or more", "at least" and "but not limited to" should not be construed to imply that the parts of the present application that do not use similar phrases intend to be limiting.

## Claims

1. A computer-implemented method for generating an orthodontic treatment plan, comprising:

   obtaining a first 3D digital model and a second 3D digital model, where the first 3D digital model represents an initial tooth arrangement of a jaw/jaws, and the second 3D digital model represents a target tooth arrangement of the jaw/jaws;
   extracting features from the first 3D digital model using a trained feature extraction deep neural network; and
   generating an orthodontic treatment plan of the jaw/jaws using a trained multi-agent reinforcement learning based deep neural network based on the extracted features and the second 3D digital model, where in the multi-agent reinforcement learning based deep neural network, each tooth of the jaw/jaws is taken as an agent, where the orthodontic treatment plan utilizes shell-shaped tooth repositioners and comprises a series of successive treatment steps to incrementally reposition the jaw/jaws from the initial tooth arrangement to the target tooth arrangement.

2. The computer-implemented method of claim 1, wherein the method further comprising: calculating displacements of single movements of the teeth based on the first 3D digital model and the second 3D digital model, the multi-agent reinforcement learning based deep neural network generating the orthodontic treatment plan based on the extracted features, the second 3D digital model and the calculated displacements of single movements of the teeth.

3. The computer-implemented method of claim 1, wherein the jaws comprise teeth on an upper jaw and a lower jaw.

4. The computer-implemented method of claim 1, wherein the method further comprising: uniformly sampling M points from each tooth of the first 3D digital model to obtain a point cloud X of the jaw/jaws, the feature extraction deep neural network extracting features from the point cloud X.

5. The computer-implemented method of claim 1, wherein the feature extraction deep neural network comprises a first MLP module and a second MLP module, where the first MLP module is configured to extract the features, and the second MLP module is configured to reconstruct the point cloud of the jaw/jaws based on the features extracted by the first MLP module, in training of the feature extraction deep neural network.

6. The computer-implemented method of claim 1, wherein the multi-agent reinforcement learning based deep neural network is one of the following networks: a recurrent network, a CommNet and a G2ANet.

7. The computer-implemented method of claim 1, wherein the multi-agent reinforcement learning based deep neural network is modeled using one of the following algorithms: Qmix, VDN, COMA and QTRAIN.

8. The computer-implemented method of claim 1, wherein in each frame, actions selectable by each agent comprise: maintaining still, translating once only, rotating once only, and translating once and rotating once.

9. The computer-implemented method of claim 1, wherein constraints imposed by the multi-agent reinforcement learning based deep neural network on the agents comprise: a constraint on displacement of a single movement, a constraint on collision, a constraint on separation of movements and a constraint on anchorage.

10. The computer-implemented method of claim 1, wherein the multi-agent reinforcement learning based deep neural network assigns a reward to each frame according to the following: whether an agent violates any constraint, whether the agent selects a translation or a rotation, whether the agent reaches its target pose, and whether all agents reach their target poses.

11. The computer-implemented method of claim 1, wherein the orthodontic treatment plan is a tooth action chart comprising an action taken by each tooth in each treatment step in the process of repositioning the jaw/jaws from the initial tooth arrangement to the target tooth arrangement.

12. A computer system for generating an orthodontic treatment plan, comprising a processor and a storage device, where the storage device stores therein a computer program for generating an orthodontic treatment plan, when executed, the computer program will cause the processor to implement the method for generating an orthodontic treatment plan of claim 1.

100

a first and a second 3D digital models are obtained — 101

features are extracted from the first 3D digital model — 103

an orthodontic treatment plan is generated by a multi-agent reinforcement learning based deep neural network based on the extracted features — 105

# FIG. 1

EP 4 661 019 A1

```
   201                        203                       205                       207
┌──────────────┐      ┌──────────────────┐     ┌──────────────┐     ┌──────────────────┐
│    Input     │      │       MLP        │     │              │     │     Output       │
│ Point Cloud  │ ───▶ │ (64, 64, 64,     │ ──▶ │  MaxPooling  │ ──▶ │  Global Feature  │
│              │      │    128, d)       │     │              │     │                  │
└──────────────┘      └──────────────────┘     └──────────────┘     └──────────────────┘
                                                                              │
   211                        209                                             │
┌──────────────────────┐  ┌──────────────────┐                               │
│       Output         │  │       MLP        │                               │
│ Reconstructed Point  │◀─│ (1024, 1024,     │◀──────────────────────────────┘
│       Cloud          │  │     M*3)         │
└──────────────────────┘  └──────────────────┘
```

**FIG. 2**

**FIG. 3**

## INTERNATIONAL SEARCH REPORT

| International application No. |
|---|
| **PCT/CN2024/070704** |

| A. | CLASSIFICATION OF SUBJECT MATTER |
|---|---|

G16H20/40(2018.01)i

According to International Patent Classification (IPC) or to both national classification and IPC

| B. | FIELDS SEARCHED |
|---|---|

Minimum documentation searched (classification system followed by classification symbols)

G16H

Documentation searched other than minimum documentation to the extent that such documents are included in the fields searched

Electronic data base consulted during the international search (name of data base and, where practicable, search terms used)

CNTXT, DWPI, VEN, ENTXT, CJFD, CNKI, IEEE, 超星读秀, DUXIU: 牙齿, 矫正, 矫治, 正畸, 方案, 计划, 布局, 神经网络, 智能体, tooth, 3D, model, correct, orthodontic, treatment, plan, intelligent, agent

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | CN 112638312 A (PROMATON HOLDING B.V.) 09 April 2021 (2021-04-09) description, paragraphs 46-88, and figures 2-12 | 1-12 |
| Y | CN 103870680 A (SHANGHAI ANGEL ALIGN MEDICAL EQUIPMENT CO., LTD.) 18 June 2014 (2014-06-18) description, paragraphs 3-18 | 1-12 |
| A | CN 113822305 A (HANGZHOU CHAOHOU INFORMATION TECHNOLOGY CO., LTD.) 21 December 2021 (2021-12-21) entire document | 1-12 |
| A | CN 115410714 A (LM TECHNOLOGY (BEIJING) CO., LTD.) 29 November 2022 (2022-11-29) entire document | 1-12 |
| A | CN 105769352 A (SHANGHAI HUIYIN INFORMATION SCIENCE AND TECHNOLOGY CO., LTD.) 20 July 2016 (2016-07-20) entire document | 1-12 |
| A | US 2021074061 A1 (ALIGN TECHNOLOGY INC.) 11 March 2021 (2021-03-11) entire document | 1-12 |

☐ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **26 February 2024** | **16 March 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2024/070704**

| Patent document cited in search report | | | Publication date (day/month/year) | Patent family member(s) | | | Publication date (day/month/year) |
|---|---|---|---|---|---|---|---|
| CN | 112638312 | A | 09 April 2021 | EP | 3846735 | A1 | 14 July 2021 |
| | | | | JP | 2021535493 | A | 16 December 2021 |
| | | | | WO | 2020048960 | A1 | 12 March 2020 |
| | | | | BR | 112021003326 | A2 | 11 May 2021 |
| | | | | US | 2021322136 | A1 | 21 October 2021 |
| | | | | CA | 3109245 | A1 | 12 March 2020 |
| | | | | IL | 281035 | A | 29 April 2021 |
| | | | | KR | 20210050562 | A | 07 May 2021 |
| | | | | EP | 3620130 | A1 | 11 March 2020 |
| | | | | JP | 2021535493 | W | 16 December 2021 |
| | | | | CN | 112638312 | B | 13 May 2022 |
| | | | | BR | 112021003326 | B1 | 13 June 2023 |
| CN | 103870680 | A | 18 June 2014 | None | | | |
| CN | 113822305 | A | 21 December 2021 | WO | 2021253917 | A1 | 23 December 2021 |
| | | | | US | 2023334771 | A1 | 19 October 2023 |
| CN | 115410714 | A | 29 November 2022 | None | | | |
| CN | 105769352 | A | 20 July 2016 | HK | 1221630 | A1 | 09 June 2017 |
| | | | | US | 2016175068 | A1 | 23 June 2016 |
| | | | | HK | 1221630 | A0 | 09 June 2017 |
| | | | | CN | 105769352 | B | 16 June 2020 |
| US | 2021074061 | A1 | 11 March 2021 | US | 2021073998 | A1 | 11 March 2021 |
| | | | | US | 11651494 | B2 | 16 May 2023 |
| | | | | US | 2024029265 | A1 | 25 January 2024 |
| | | | | US | 11232573 | B2 | 25 January 2022 |

Form PCT/ISA/210 (patent family annex) (July 2022)